(19) Europäisches Patentamt
European Patent Office
Office européen des brevets

(11) Veröffentlichungsnummer: **0 216 325 A2**

(12) **EUROPÄISCHE PATENTANMELDUNG**

(21) Anmeldenummer: 86112952.6

(51) Int. Cl.⁴: C07D 207/38

(22) Anmeldetag: 19.09.86

(30) Priorität: 24.09.85 CH 4126/85
20.05.86 CH 2023/85

(43) Veröffentlichungstag der Anmeldung:
01.04.87 Patentblatt 87/14

(84) Benannte Vertragsstaaten:
AT BE CH DE FR GB IT LI LU NL SE

(71) Anmelder: LONZA AG

Gampel/Wallis(CH)

(72) Erfinder: Meul, Thomas, Dr.
Balfrinstrasse 21
Visp Kanton Wallis(CH)
Erfinder: McGarrity, John, Dr.
Rathausstrasse 5
Visp Kanton Wallis(CH)

(74) Vertreter: Weinhold, Peter, Dr. et al
Patentanwälte Dipl.-Ing. G. Dannenberg Dr.
P. Weinhold Dr. D. Gudel Dipl.-Ing. S.
Schubert Dr. P. Barz Siegfriedstrasse 8
D-8000 München 40(DE)

(54) **Verfahren zur Herstellung von 4-Hydroxy-2-oxo-pyrrolidin-1-yl-acetamid.**

(57) Zur Herstellung des cerebral wirksamen 4-Hydroxy-2-oxo-pyrrolidin-1-yl-acetamids wird ein neues einfaches Verfahren ausgehend von 4-Alkoxy-3-pyrrolin-2-on-1-yl-essigsäurealkylestern beschrieben.

EP 0 216 325 A2

Xerox Copy Centre

# Verfahren zur Herstellung von 4-Hydroxy-2-oxo-pyrrolidin-1-yl-acetamid

Die Erfindung betrifft ein neues Verfahren zur Herstellung des cerebral wirksamen 4-Hydroxy-2-oxo-pyrrolidin-1-yl-acetamids. Es ist aus Pifferi et al., Il Farmaco, Ed.Sc., 1977, 32, 602 bekannt, 4-Hydroxy-2-oxo-pyrrolidin-1-yl-acetamid über 5 Stufen herzustellen. Teure Ausgangsprodukte und eine Gesamtausbeute um 33.8 % machen dieses Verfahren jedoch nicht rentabel. Es stellt sich daher die Aufgabe, einen Weg zu finden, der die genannten Nachteile nicht aufweist.

Gelöst wurde die Aufgabe erfindungsgemäss durch ein Verfahren nach Anspruch 1. Darin wird ein 4-($C_1$-$C_2$)-alkoxy-3-pyrrolin-2-on-1-yl-essigsäure-($C_1$-$C_4$)-alkylester, vorzugsweise 4-Methoxy-3-pyrrolin -2-on-1-yl-essigsäureethylester entweder mit Trichlormethylsilan in Gegenwart eines Alkalijodids, vorzugsweise mit Natriumjodid, oder in einem sauren wasserfreien Medium umgesetzt.

Wählt man die Umsetzung mit Trichlormethylsilan und Alkalijodid, wird zweckmässig so vorgegangen, dass man die Edukte 4-($C_1$-$C_2$)-alkoxy-3-pyrrolin -2-on-1-yl-essigsäure-($C_1$-$C_4$)-alkylester, Trichlormethylsilan und Alkalijodid in einem Molverhältnis zwischen zweckmässig 1 zu 1 zu 1 und 1 zu 2 zu 2, vorzugsweise zwischen 1 zu 1,2 zu 1,2 und 1 zu 1,6 zu 1,6 einsetzt.

Vorteilhaft wird diese Umsetzung in Acetonitril als Lösungsmittel durchgeführt.

Die Umsetzungstemperatur liegt bevorzugt im Bereich der Rückflusstemperatur des Lösungsmittels.

Nach einer Reaktionszeit von ca. 1 bis 5 Stunden und üblicher Aufarbeitung durch z.B. Extraktion und gegebenenfalls durch anschliessende Reinigung des Produktes durch z.B. Umkristallisation kann der entsprechende 2,4-Dioxo-pyrrolidin-1-yl-essigsäure-($C_1$-$C_4$)-alkylester erhalten werden.

Verfährt man nach der zweiten Variante in einem sauren wasserfreien Medium, wird zweckmässig folgendermassen vorgegangen. Das saure Medium wird zweckmässig durch Einleiten von gasförmiger Salzsäure oder gasförmigem Bromwasserstoff, in eine $C_1$-$C_4$-Carbonsäure, die ebenfalls wasserfrei ist, erzeugt. Bevorzugte $C_1$-$C_4$-Carbonsäure ist die Essigsäure. Besonders bevorzugt wird gasförmige Salzsäure in Essigsäure verwendet. Die Umsetzungstemperatur wählt man zweckmässig zwischen 0 und 50°C.

Nach einer Reaktionszeit von 5 bis 10 Stunden kann das Reaktionsgemisch auf übliche Weise durch z.B. Abdampfen des Lösungsmittels und gegebenenfalls durch Kristallisation aufgearbeitet werden.

Die Ueberführung der 2,4-Dioxo-pyrrolidin-1-yl-essigsäure-($C_1$-$C_4$)-alkylester, speziell des Ethylesters, in das Zielmolekül 4-Hydroxy-2-oxo-pyrrolidin-1-yl-acetamid durch Reduktion mit Natriumborhydrid und nachfolgende Amidierung mit Ammoniak wurde in der Literatur bereits vorexerziert.

(G. Pifferi, M. Pinza, Il Farmaco, Ed.Sc., 1977, 32, 602.) Das 4-Hydroxy-2-oxo-pyrrolidin-1-yl-acetamid kann danach als reines weisses Produkt in guter Ausbeute erhalten werden.

## Beispiel 1

### a) Herstellung von 2,4-Dioxo-pyrrolidin-2-on-1-yl-essigsäureethylester

2,0 g (10 mmol) 4-Methoxy-3-pyrrolin-2-on-1-yl-essigsäureethylester und 2,1 g (14 mmol) Natriumjodid wurden in 20,0 ml Acetonitril gelöst. Anschliessend gab man 1,6 g (14 mmol) Methyltrichlorsilan zu. Die gelblich gefärbte trübe Reaktionslösung wurde 4 Stunden unter Rückfluss erhitzt. Man liess auf Raumtemperatur abkühlen und dampfte das Lösungsmittel unter Wasserstrahlvakuum ab. Der Rückstand wurde in 50,0 ml Methylenchlorid aufgeschlämmt und mit einer Lösung aus 1,0 g Natriumbisulfat in 5,0 ml Eiswasser versetzt. Die wässrige Phase wurde dreimal mit je 50,0 ml Methylenchlorid extrahiert. Die vereinigten organischen Phasen wurden über $Na_2SO_4$ getrocknet, eingedampft und am Hochvakuum getrocknet.

Man erhielt 1,9 g einer orangegefärbten Masse, die langsam durchkristallisierte.

Das umkristallisierte Produkt (Toluol:Petrolether 1:1) schmolz bei 92 bis 93°C.

NMR ($CDCl_3$) $\delta$ = 4,25 (s, 2H), 4,23 (q, J=7,1 Hz, 2H);

4,03 (t, J=1,0 Hz, 2H); 3,11 (br. s, 2H);

1,30 (t, J=7,1 Hz, 3H).

### b) Herstellung von 4-Hydroxy-2-oxo-pyrrolidin-1-yl-essigsäureethylester

Zu 8,8 g (47,5 mmol) 2,4-Dioxo-pyrrolidin-1-yl-essigsäureethylester in 200 ml Dimethoxyethan wurden bei 0°C 0,54 g (14,5 mmol) Natriumborhydrid zugegeben. Die Mischung wurde während einer Stunde bei Raumtemperatur gerührt. Nach

Abkühlen auf 0°C wurde konzentrierte Salzsäure im Ueberschuss zugegeben und der anorganische Rückstand abfiltriert. Das Filtrat wurde zur Trockene eingedampft. Der Rückstand wurde in Chloroform aufgenommen, getrocknet über $Na_2SO_4$ und erneut unter Vakuum eingedampft.

Es resultierten 8,8 g des Produktes in öliger Form.

Eine anschliessende Destillation bei 180°C/1,0 mbar führte zu 5,3 g (60%) eines farblosen Oeles.

IR (Film) 3400 $cm^{-1}$ (OH), 1740 $cm^{-1}$ (C=O), 1680 $cm^{-1}$ (C=O),

1200 $cm^{-1}$.

c) Herstellung von 4-Hydroxy-2-oxo-pyrrolidin-1-yl-acetamid

13,7 g (73 mmol) 4-Hydroxy-2-oxo-pyrrolidin-1-yl-essigsäureethylester wurden gelöst in 500 ml Methanol und bei 0°C mit Ammoniak gesättigt. Danach wurde der Ansatz während einer Stunde bei Raumtemperatur gehalten. Abdampfen des Lösungsmittels unter Vakuum führte zu einem festen Rückstand. Dieser wurde in Methanol kristallisiert.

Es resultierten 7,17 g (62%) des Produktes in Form eines weissen Pulvers. Fp 165 bis 168°C.

IR (Nujol) 3400 $cm^{-1}$, 3300 $cm^{-1}$, 3250 $cm^{-1}$ (OH und NH)

1660 $cm^{-1}$ (C=O).

Beispiel 2

a) Herstellung von 2,4-Dioxo-pyrrolidin-1-yl-essigsäureethylester

10,0 g 4-Methoxy-3-pyrrolin-2-on-1-yl-essigsäureethylester (GC-Gehalt: 80 %) wurden in 50,0 ml Essigsäure gelöst und bei 35 bis 40°C 9 Stunden unter Rühren mit gasförmiger Salzsäure gesättigt. Anschliessend wurde die Essigsäure am Rotationsverdampfer unter Vakuum bei einer Badtemperatur von 60°C eingedampft. Der Rückstand wurde in 50,0 ml Toluol aufgenommen und nochmals eingedampft. Als Rückstand verblieben 12,4 g Rohprodukt, das im Kühlschrank kristallisierte.

b) Herstellung von 4-Hydroxy-2-oxo-pyrrolidin-1-yl-essigsäureethylester

12,2 g des oben erhaltenen Rohproduktes wurden in 50,0 ml Acetonitril gelöst und bei Raumtemperatur zu einer Suspension von 1,6 g Natriumborhydrid in 50,0 ml Acetonitril gegeben. Dabei stieg die Reaktionstemperatur auf 35 bis 40°C. Bei dieser Temperatur wurde die Reaktionslösung eine Stunde nachgerührt. Anschliessend wurde mit konzentrierter Salzsäure angesäuert auf einen pH von ca. 5. Die Lösung wurde am Rotationsverdampfer eingeengt, der Rückstand mit 25,0 ml Eiswasser versetzt und mit Methylenchlorid extrahiert. Nach Trocknen der Methylenchlorid-Lösung über Natriumsulfat und Eindampfen des Lösungsmittels verblieben 9,5 g Rohprodukt mit einem Gehalt nach HPLC von 51,5 %. Dies entsprach einer Ausbeute von 66,1 % bezogen auf eingesetztes 4-Methoxy-3-pyrrolin-2-on-1-yl-essigsäureethylester.

Chromatographische Filtration über Kieselgel mit Essigester als Laufmittel ergab ein Produkt mit einem Gehalt von grösser 96 % (HPLC).

Die Herstellung des 4-Hydroxy-2-oxo-pyrrolidin-1-yl-acetamids wurde entsprechend 1 c) durchgeführt.

**Ansprüche**

1. Verfahren zu Herstellung von 4-Hydroxy-2-oxo-pyrrolidin-1-yl-acetamid, dadurch gekennzeichnet, dass man 4-($C_1$-$C_2$) alkoxy-3-pyrrolin-2-on-1-yl-essigsäure-($C_1$-$C_4$)-alkylester der Formel

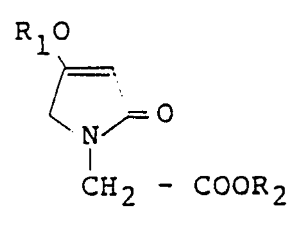

worin $R_1$ = Alkyl mit 1 bis 2 C-Atomen und $R_2$ = Alkyl mit 1 bis 4 C-Atomen bedeutet, entweder mit Trichlormethylsilan in Gegenwart eines Alkalijodids oder in saurem wasserfreien Medium zum 2,4-Dioxo-pyrrolidin-1-yl-essigsäure-($C_1$-$C_4$)-alkylester umsetzt, diesen gegebenenfalls isoliert und anschliessend mit Natriumborhydrid zu einem 4-Hydroxy-2-oxo-pyrrolidin-1-yl-essigsäure-$C_1$-$C_4$-alkylester hydriert und schliesslich durch Amidierung mittels Ammoniak den 4-Hydroxy-2-oxo-pyrrolidin-1-yl-essigsäure-$C_1$-$C_4$-alkylester in das gewünschte Endprodukt überführt.

2. Verfahren gemäss Patentanspruch l, dadurch gekennzeichnet, dass als Alkalijodid Natriumjodid verwendet wird.

3. Verfahren gemäss Patentansprüchen 1 und 2, dadurch gekennzeichnet, dass die Umsetzung mit Trichlormethylsilan in Gegenwart eines Alkalijodids zusätzlich in Gegenwart von Acetonitril als Lösungsmittel erfolgt.

4. Verfahren gemäss Patentanspruch 1, dadurch gekennzeichnet, dass das saure wasserfreie Medium durch Einleiten von gasförmiger Salzsäure oder gasförmigem Bromwasserstoff in eine wasserfreie $C_1$-$C_4$-Carbonsäure erzeugt wird.

5. Verfahren gemäss Patentansprüchen 1 und 4, dadurch gekennzeichnet, dass als wasserfreie $C_1$-$C_4$-Carbonsäure Essigsäure verwendet wird.

6. Verfahren gemäss Patentanprüchen 1, 4 und 5, dadurch gekennzeichnet, dass mit gasförmiger Salzsäure in wasserfreier Essigsäure gearbeitet wird.